(19) Europäisches Patentamt
European Patent Office
Office européen des brevets

(11) Publication number: 0 061 001
A1

(12) EUROPEAN PATENT APPLICATION

(21) Application number: 82101303.4

(22) Date of filing: 19.02.82

(51) Int. Cl.³: A 61 K 31/70
//C07H19/16, C07H19/20

(30) Priority: 24.02.81 JP 26560/81

(43) Date of publication of application:
29.09.82 Bulletin 82/39

(84) Designated Contracting States:
BE CH DE FR GB IT LI NL SE

(71) Applicant: Yamasa Shoyu Kabushiki Kaisha
10-1, Araoi-Cho 2-Chome
Choshi-Shi Chiba-Ken(JP)

(72) Inventor: Ichikawa, Masatsune
2-2, Sakae-Cho 2-Chome
Choshi-Shi Chiba-Ken(JP)

(72) Inventor: Fujiyama, Kazuo
2-2, Sakae-Cho 2-Chome Choshi-Shi
Chiba-Ken(JP)

(72) Inventor: Shibuya, Susumu
359, South Ridge, 45 Avenue 106 Street
Edmonton Alberta, T6H 4M9(CA)

(72) Inventor: Ikeda, Takao
4-201, Daigo Ishida Danchi 3, Ishida Sakuragi-Cho
Fushimi-Ku Kyoto-Shi Kyoto-Fu(JP)

(74) Representative: Dr. Elisabeth Jung Dr. Jürgen Schirdewahn Dr. Gerhard Schmitt-Nilson Dr. Gerhard B. Hagen Dipl.-Ing. Peter Hirsch
P.O. Box 40 14 68
D-8000 München 40(DE)

(54) **Antiallergic agent comprising derivatives of adenosine.**

(57) Antiallergic effect is exhibited by an $N^6$-ω-aminoalkyl adenosine compound of the formula:

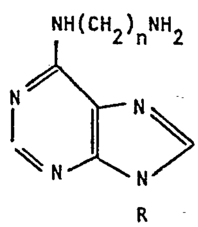

wherein n is an integer of 1 to 20, and R is a β-D-ribofuranosyl group represented by the formula

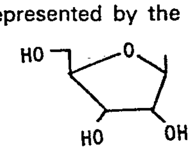

;

a 5-phosphoryl-β-D-ribofuranosyl group of the formula

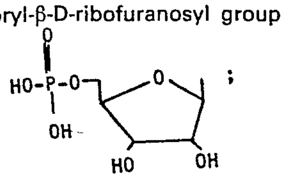

;

a 3-phosphoryl-β-D-ribofuranosyl group of the formula

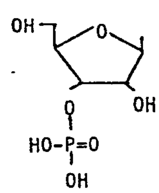

; or

a 3,5-cyclicphosphoryl-β-D-ribofuranosyl group of the formula

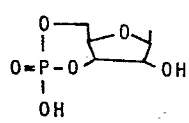

or a pharmaceutically acceptable salt thereof.

EP 0 061 001 A1

YAMASA SHOYU KABUSHIKI KAISHA
10-1, ARAOI-CHO 2-CHOME, CHOSHI-SHI,
CHIBA-KEN, JAPAN



0061001 Feb. 9, 1982
Our Ref.: Q 918 (Dr.J/su)

ANTIALLERGIC

TITLE MODIFIED
see front page

FIELD OF THE ART

This invention relates to an antiallergic, comprising an $N^6$-ω-aminoalkyl adenosine compound or a pharmaceutically acceptable salt thereof as an effective ingredient, and a method for treating antiallergic disorder by using the same.

BACKGROUND OF THE ART

The compound of the present invention is a drug exhibiting an antiallergic effect by inhibiting release of a mediator from mast cells. In the prior art, as an antiallergic of this type, there has been known disodium cromoglycate (DSCG). Its effect, however, is not very marked.

The compound of the present invention is also a nucleic acid related compound having a purine nucleus. In the prior art, as an antiallergic having a purine nucleus, there have been known xanthine derivatives such as aminophylline. But many of these derivatives may exhibit undesirable side-effects such as central nervous system stimulating effect.

We have carried out screening of newly synthesized nucleic acid related compounds with the intention of obtaining compounds having more potent antiallergic effect with less side effects than the above compounds of the prior art, by utilizing the reaction of passive cutaneous

anaphylaxis (hereinafter referred to as PCA : See, for example, "Kabinshō No Sokuteihō" (Assay of Hypersensitivity) written by Tomio Tada, in "Ikagaku Jikken-hō Kōza" (Course of Medicochemical Experimental Methods) edited by Shunsuke Migita, Vol. 4 "Men'eki Kagaku" (Immunochemistry), page 235, published by Nakayama Shoten, Japan, 1972), which has been well known as a very good means for the study of allergic reaction. As a result, it has been found that the $N^6$-aminoalkyl adenosine compounds and salts thereof have remarkably potent anti-PCA activity and are useful for therapy or prevention of various allergic diseases. The present invention has been achieved on the basis of this and related findings.

## SUMMARY OF THE INVENTION

The present invention provides an antiallergic, comprising an $N^6$-ω-aminoalkyl adenosine compound of the formula (I):

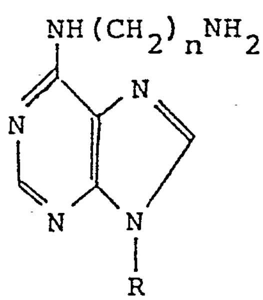

(I)

or a pharmaceutically acceptable salt thereof as an effective ingredient.

In the formula (I), n is an integer of 1 to 20, and R is a ribose residue or a phosphoryl ribose residue as shown below:

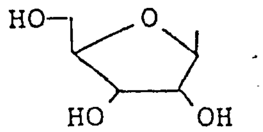

β-D-Ribofuranosyl group,

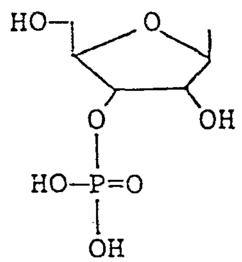

3-Phosphoryl-β-D-ribofuranosyl group,

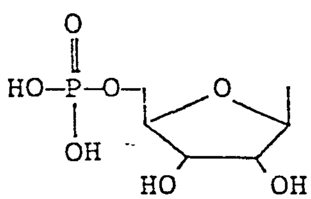

5-Phosphoryl-β-D-ribofuranosyl group, or

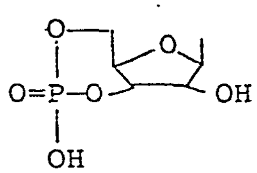

3,5-Cyclicphosphoryl-β-D-ribofuranosyl group.

## DETAILED DESCRIPTION OF THE INVENTION

More specifically, the compounds of the present invention are inclusive of $N^6$-ω-aminoalkyl adenosine (hereinafter abbreviated as $N^6$-ω-aminoalkyl Ado), $N^6$-ω-aminoalkyl adenosine-5'-monophosphate (hereinafter abbreviated as $N^6$-aminoalkyl-5'-AMP), $N^6$-ω-aminoalkyl adenosine-3'-monophosphate (hereinafter abbreviated as $N^6$-ω-aminoalkyl-3'-AMP) and $N^6$-ω-aminoalkyl adenosine-3',5'-cyclic monophosphate (hereinafter abbreviated as $N^6$-ω-aminoalkyl-cAMP), and pharmaceutically acceptable salts thereof.

Typical examples of $N^6$-ω-aminoalkyl Ado include the following.

$N^6$-(2-aminoethyl)-Ado

$N^6$-(3-amino-n-propyl)-Ado

$N^6$-(4-amino-n-butyl)-Ado

$N^6$-(5-amino-n-pentyl)-Ado

$N^6$-(6-amino-n-hexyl)-Ado

$N^6$-(7-amino-n-heptyl)-Ado

$N^6$-(8-amino-n-octyl)-Ado

$N^6$-(9-amino-n-nonyl)-Ado

$N^6$-(10-amino-n-decanyl)-Ado

$N^6$-(12-amino-n-dodecanyl)-Ado

$N^6$-(16-amino-n-hexadecanyl)-Ado

$N^6$-(18-amino-n-octadecanyl)-Ado

Also as typical examples of $N^6$-ω-aminoalkyl-5'-AMP, $N^6$-ω-aminoalkyl-3'-AMP and $N^6$-ω-aminoalkyl-cAMP, there may be mentioned 5'-monophosphate, 3'-monophosphate or 3', 5'-cyclic monophosphate corresponding to the above $N^6$-ω-aminoalkyl-Ado.

Among the above compounds, $N^6$-ω-aminoalkyl Ado wherein n = 6 or more, $N^6$-ω-aminoalkyl-5'-AMP wherein n = 13 or more, and all of $N^6$-ω-aminoalkyl-3'-AMP and $N^6$-ω-aminoalkyl-cAMP are novel compounds.

The salts of these compounds are not particularly limited as long as they are pharmaceutically acceptable. For example, salts with alkali metals such as sodium, potassium, lithium, etc.; with alkaline earth metals such as calcium; with ammonia; with mineral acids such as hydrochloric acid, sulfuric acid, etc.; with organic acids

such as acetic acid; and with amino acids may be employed.

The compounds according to the present invention have antiallergic activity and are effective for therapy or prevention of diseases such as allergic asthma,hay asthma, allergic conjunctivitis, allergic rhinitis, allergic dermatitis, etc.

The compounds of the present invention can be formulated into any desired preparation depending on the route of administration according to conventional methods such as suspensions, aerosols, inhalants, powders, tablets, fine powders, capsules, granules, pills, syrups, tinctures, injections, suppositories, ointments, cataplasms, and others.

In making preparations, in the case of liquid preparations, conventionally used additives such as emulsifiers, suspension aids, preservatives, stabilizers, resolvents, etc. may suitably be used. In the case of solid preparations, suitable use may be made of conventional binders, excipients, lubricants, disintegraters, etc. If necessary, there may also be added corrigents or edulcorants.

The compounds of the present invention may be administered according to any desired route suitably selected depending on the compound employed and its dosage from oral application, inhalation, dermal and mucosal application, intravenous injection, intramuscular injection or intrarectal administration.

The compounds of the present invention or salts thereof may be administered in a dosage, which differs depending on the compound employed, dosage form or age, body weight or severity of disease of the patient, but the dosage per day is ordinarily in the range of from 0.01 to 10 mg/kg-body weight.

Referring now to the antiallergic activity of $N^6$-ω-aminoalkyl adenosine compounds of the present invention, the test results obtained for anti-PCA action are set forth below.

Experimental Example 1

Passive cutaneous anaphylaxis (PCA) suppressing effect

According to the method of J. Goose et al (see Immunology 16, 749, 1969), PCA reaction was carried out in the following manner.

To male rats (Sprague Dawley-strain; hereinafter abbreviated as SD-strain), weighing 120 to 150 g, 10 mg/kg of ovalubumin was administered intramuscularly and 2 x $10^{10}$ cells of *Bodetalla pertussis* vaccine intraperitoneally. After 10 to 12 days, each rat was bled and antiserum was separated.

To other rats (SD-strain, weighing 120 to 150 g) shaved at the back, under ether anesthetization, 0.1 ml of the above antiserum diluted 4-fold was injected intradermally for dermal sensitization. After 48 hours, 1 mg/kg of ovalubumin (antigen), 1 mg/kg of Evans blue (dye) and various $N^6$-ω-aminoalkyl adenosine compounds

and DSCG or isotonic saline were challenged intravenously. After 40 minutes, the diameter of dye spots on the skin and the leak quantity of dye were measured to determine $ED_{50}$ (50% effective amount) for each compound.

The leak quantity of dye was determined by extracting the dye from the skin peeled off with potassium hydroxide, removing proteins with acetone after neutralization with phosphoric acid, and thereafter measuring absorbance at 620 nm of the resultant extract, which step was then followed by calculation therefrom. The $N^6$-ω-aminoalkyl adenosine compounds provided for the tests are enumerated below:

| Sample No. | Name of Compound |
|---|---|
| 1 | $N^6$-(3-amino-n-propyl)-Ado |
| 2 | $N^6$-(6-amino-n-hexyl)-Ado |
| 3 | $N^6$-(7-amino-n-heptyl)-Ado |
| 4 | $N^6$-(9-amino-n-nonyl)-Ado |
| 5 | $N^6$-(3-amino-n-propyl)-5'-AMP |
| 6 | $N^6$-(6-amino-n-hexyl)-5'-AMP |
| 7 | $N^6$-(9-amino-n-nonyl)-5'-AMP |
| 8 | $N^6$-(3-amino-n-butyl)-3'-AMP |
| 9 | $N^6$-(6-amino-n-hexyl)-3'-AMP |
| 10 | $N^6$-(8-amino-n-octyl)-3'-AMP |
| 11 | $N^6$-(4-amino-n-butyl)-cAMP |
| 12 | $N^6$-(6-amino-n-hexyl)-cAMP |
| 13 | $N^6$-(8-amino-n-octyl)-cAMP |

| | |
|---|---|
| 14 | $N^6$-(9-amino-n-nonyl)-cAMP |
| 15 | $N^6$-(10-amino-n-decanyl)-cAMP |

Table 1

| Sample No. | $ED_{50}$ (mg/kg) | |
|---|---|---|
| | By diameter | By dye quantity |
| 1 | 0.87 | 0.20 |
| 2 | 0.15 | 0.06 |
| 3 | 0.058 | 0.06 |
| 4 | 0.084 | 0.084 |
| 5 | 0.46 | 0.31 |
| 6 | 0.51 | 0.32 |
| 7 | 0.29 | 0.16 |
| 8 | 1.7 | 0.64 |
| 9 | 1.9 | 0.70 |
| 10 | 1.5 | 0.70 |
| 11 | 15.0 | 9.4 |
| 12 | 3.3 | 3.2 |
| 13 | 3.7 | 3.1 |
| 14 | >3.3 | 3.3 |
| 15 | 8.2 | 6.0 |
| DSCG | 3.3 | 3.0 |

Experimental Example 2

Acute toxicity tests

To ICR-JCL-strain mice (male, weighing 24 to 28 g),

various $N^6$-ω-aminoalkyl adenosine compounds (each in free form) were administered as isotonic saline solutions or suspensions each in a dosage of 0.1 ml/10 g intraperitoneally for determination of $LD_{50}$. The results obtained are shown below.

| Name of Compound | $LD_{50}$ (mg/kg) |
|---|---|
| $N^6$-(7-amino-n-heptyl)-Ado | 145(138 - 152)* |
| $N^6$-(9-amino-n-nonyl)-Ado | 175(166 - 184) |
| $N^6$-(9-amino-n-nonyl)-5'-AMP | 195(173 - 217) |
| $N^6$-(8-amino-n-octyl)-3'-AMP | 220(200 - 240) |
| $N^6$-(6-amino-n-hexyl)-cAMP | 165(150 - 180) |

*95% confidence limit

The $N^6$-ω-aminoalkyl adenosine compounds which are the active compounds in the present invention can be prepared according to any suitable process which is not particularly limited. Typical processes for preparations of the compounds are described below.

The starting compounds are selected, depending on the sugar residues of the desired compounds from 6-halogenopurinenucleoside compounds of the formula (II) shown below or salts thereof suitable for the synthetic reactions.

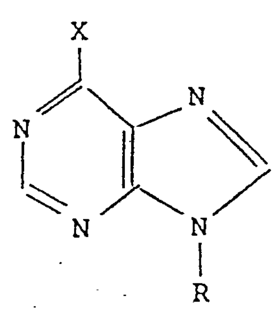

(II)

wherein R is a β-D-ribofuranosyl group represented by the formula:

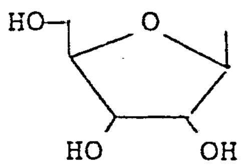

,

a 5-phosphoryl-β-D-ribofuranosyl group of the formula:

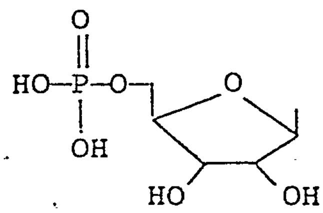

,

a 3-phosphoryl-β-D-ribofuranosyl group of the formula:

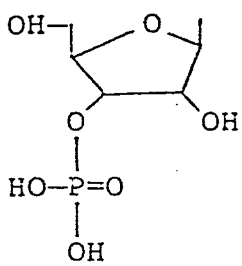

,

or a 3,5-cyclicphosphoryl-β-D-ribofuranosyl of the formula:

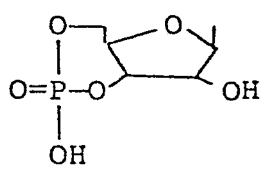

and X represents a halogen atom.

The desired compounds can be obtained by causing 1,ω-diamino alkane of the following formula (III) corresponding to the $N^6$-aminoalkyl groups of the desired compounds to react with the starting compounds.

$$H_2N(CH_2)_nNH_2 \qquad (III)$$

wherein n is an integer of 1 to 20.

Such a process for preparation of $N^6$-ω-aminoalkyl cAMP has been disclosed in Japanese Patent Application No. 108380/1979. It is also possible to obtain $N^6$-ω-aminoalkyl-3'-AMP as a side product when 6-halogeno-9-β-D-ribofuranosylpurine 3',5'-cyclic phosphate is reacted with a 1,ω-diaminoalkane (as disclosed in Japanese Patent Application No. 108381/1979).

Typical examples of the starting compounds are 6-chloro-9-β-D-ribofuranosylpurine (hereinafter abbreviated as CRP), 6-chloro-9-β-D-ribofuranosylpurine 5'-phosphate (hereinafter abbreviated as CRP-5'-P), 6-chloro-9-β-D-ribofuranosylpurine 3'-phosphate, and 6-chloro-9-β-D-ribofuranosylpurine 3',5'-cyclicphosphate (hereinafter abbreviated as CRP-3',5'-P).

Typical examples of 1,ω-diaminoalkane are ethylenediamine, 1,3-diamino-n-propane, 1,4-diamino-n-butane, 1,5-diamino-n-pentane, 1,6-diamino-n-hexane, 1,7-diamino-n-heptane, 1,8-diamino-n-octane, 1,9-diamino-n-nonane, 1,10-diamino-n-decane, 1,12-diamino-n-dodecane, 1,16-diamino-n-hexadecane, 1,18-diamino-n-octadecane and the like.

The reaction may be carried out in a solvent which is not specifically limited. Examples of the solvent are water, ethanol, methanol, dimethylformamide, dimethylacetamide, dimethyl sulfoxide, and pyridine.

The reaction conditions may suitably be selected depending on the desired compound, the reactants and the solvent employed. The reaction is carried out ordinarily at a temperature in the range from room temperature to 100°C and completed within 1 to a number of tens of hours.

After completion of the reaction, the desired compound can be purified and isolated according to any suitable method which is not particularly limited, but the methods conventionally used for purification of organic compounds can be applied. For example, such methods as partition method, recrystallization method, partition chromatography, absorption chromatography, ion-exchange chromatography, etc. may be suitably selected or combined. The salt conversion, salt exchange or desalting of the desired compound may also be performed according to conventional methods.

The present invention will now be described in specific detail by way of preparation examples of typical exemplary $N^6$-ω-aminoalkyl adenosine compounds of the present invention.

Preparation Example 1

Synthesis of $N^6$-(3-amino-n-propyl)-Ado

In 100 ml of dimethylformamide, 5 g of CRP and 15 ml of 1,3-diamino-n-propane were dissolved, and the reaction was carried out at room temperature for 3 hours. The reaction mixture was concentrated under reduced pressure, and the

residue was dissolved in water. Then, the solution was adjusted to pH 10 with 1 N sodium hydroxide, and white precipitates were collected and recrystallized from ethanol to obtain 3 g of $N^6$-(3-amino-n-propyl)-Ado.

m.p. 192 - 193°C

Preparation Example 2

Synthesis of $N^6$-(6-amino-n-hexyl)-Ado

Into 20 ml of a dimethylformamide solution containing 4.64 g of 1,6-diamino-n-hexane and 1.4 ml of triethylamine dissolved therein was added dropwise under stirring 20 ml of a dimethylformamide solution having 2.87 g of CRP dissolved therein, and the reaction was carried out at 40°C for 3 hours. The reaction mixture was concentrated under reduced pressure, and the residue was neutralized with hydrochloric acid. The resultant solution was concentrated to dryness under reduced pressure and then dissolved in dimethylformamide. After insolubles were filtered out, the dimethylformamide was evaporated under reduced pressure, and the residue was dissolved in water. The solution was then cooled overnight, and the insolubles were filtered out. The filtrate was applied to a column of 100 ml of Dowex 50 x 4 (free acid form, produced by Dow Chemical Co.) and, after washing with water and 0.3% aqueous ammonia, the column was eluted successively with three mixtures of 5% aqueous ammonia and ethanol respectively in ratios of 9:1, 4:1 and 7:3. The eluate was concentrated

and recrystallized from water to produce 1.69 g of $N^6$-(6-amino-n-hexyl)-Ado (Yield : 44.9%).

Elemental analysis for $C_{16}H_{26}N_6O_4 \cdot 1/2H_2O$

Calculated : C, 51.19; H, 7.25: N, 22.39

Measured : C, 51.11; H, 7.08; N, 22.15

UV absorption spectrum $\lambda_{max}^{MeOH}$ 267 nm

Preparation Example 3

Synthesis of $N^6$-(7-amino-n-heptyl)-Ado

A dimethylformamide solution containing 2.3 g of CRP was added dropwise under stirring into a dimethylformamide solution containing 5 g of 1,7-diamino-n-heptane and 1.2 ml of triethylamine, and the reaction was carried out at 50°C for 8 hours. Following the same procedure as in Preparation Example 2, the solution of the reaction product was applied to a column of Dowex 50 x 4 (free acid form) and, after washing with 1% aqueous ammonia, elution was carried out using a linear gradient of 1% aqueous ammonia → 5% aqueous ammonia/30% ethanol. The eluted fractions containing the desired product were collected, concentrated, and recrystallized from water, whereupon 1.1 g of $N^6$-(7-amino-n-heptyl)-Ado (Yield: 36.2%) was obtained.

UV absorption spectrum $\lambda_{max}^{MeOH}$ 267 nm

Preparation Example 4

Synthesis of $N^6$-(9-amino-n-nonyl)-Ado

A dimethylformamide solution (20 ml) containing 2.3 g

of CRP was added dropwise under stirring into an aqueous solution containing 5 g of 1,9-diamino-n-nonane and 1.2 ml of triethylamine, and the reaction was carried out at 50°C for 2 hours. Following the same procedure as in Preparation Example 2, the solution of the reaction product was applied to a column of Dowex 50 x 4 (free acid form) and, after washing with 5% aqueous ammonia and with water, elution was carried out using a linear gradient of ammonia (0 → 5%) in 30% ethanol. The eluted fractions containing the desired product were collected, concentrated, and then recrystallized from water, whereupon 2.0g of $N^6$-(9-amino-n-nonyl)-Ado (Yield: 61.3%) was obtained.

UV absorption spectrum $\lambda_{max}^{MeOH}$ 267 nm

Preparation Example 5

Synthesis of $N^6$-(3-amino-n-propyl)-5'-AMP

In 100 ml of water, 3g of CRP-5'-P and 10 ml of 1,3-diamino-n-propane were dissolved, and the reaction was carried out at room temperature for 3 hours. The reaction mixture was concentrated, applied to a column of Amberlite IRA-402 (acetic acid form: Rohm & Haas Co.), and eluted using a linear gradient of acetic acid (0 → 1 M). The eluted fractions containing the desired product were collected and concentrated. The precipitated crystals were collected by filtration and washed with water to produce 1.5 g of $N^6$-(3-amino-n-propyl)-5'-AMP.

UV absorption spectrum $\lambda_{max}^{H_2O}$ 268 nm

$\lambda_{max}^{H_3O^+}$ 263 nm

Preparation Example 6

Synthesis of $N^6$-(6-amino-n-hexyl)-5'-AMP

In 100 ml of water, 3 g of CRP-5'-P and 10 ml of 1,6-diamino-n-hexane were dissolved, and the reaction was carried out at room temperature for 15 hours. The reaction mixture was concentrated, applied to a column of Amberlite IRA-402 (acetic acid form: Rohm & Haas Co.), and eluted with 0.5M acetic acid. Acetic acid was evaporated off from the eluate while some additional water was added thereto. The thus precipitated crystals were collected by filtration and washed with acetone and ether to produce 2.5g of $N^6$-(6-amino-n-hexyl)-5'-AMP.

UV absorption spectrum $\lambda_{max}^{H_2O}$ 268 nm

$\lambda_{max}^{H_3O^+}$ 263 nm

Preparation Example 7

Synthesis of $N^6$-(9-amino-n-nonyl)-5'-AMP

In 100 ml of water, 3g of CRP-5'-P and 10 ml of 1,9-diamino-n-nonane were dissolved, and the reaction was carried out at room temperature for 3 hours. The reaction mixture was concentrated, adsorbed on a column of activated charcoal and, after washing with water,

eluted with 50% ethanol - 2% aqueous ammonia, which step was then followed by concentration of the eluate. The concentrate was applied to a column of Amberlite IRA-402 and eluted using a linear gradient of acetic acid (0 → 1 M). The eluted fractions containing the desired product were collected and concentrated. The precipitated crystals were collected by filtration and washed with water, whereupon 1.3 g of $N^6$-(9-amino-n-nonyl)-5'-AMP was obtained.

UV absorption spectrum $\lambda_{max}^{H_2O}$ 268 nm

$\lambda_{max}^{H_3O^+}$ 263 nm

Preparation Example 8

Synthesis of $N^6$-(4-amino-n-butyl)-3'-AMP

In 100 ml of water, 5.2 g of CRP-3', 5'-P, 24 g of 1,4-diamino-n-butane and 3 ml of triethylamine were dissolved, and the reaction was carried out at 70 to 80°C for 3 hours. The reaction mixture was diluted with water, then made acidic with hydrochloric acid and applied to a column of activated charcoal. Then, after washing with water, the column was eluted with a mixture of ethanol: conc. aqueous ammonia: water (50 : 48 : 2) and the eluate was developed through a column of Dowex 1 x 2 (formic acid form). After washing with water, the column was eluted using a linear gradient of formic acid (0 → 1 M). The eluted fractions

containing the desired product were collected, concentrated and crystallized from water-ethanol, whereupon 1.3 g of $N^6$-(4-amino-n-butyl)-3'-AMP (Yield: 21%) was obtained.

m.p. 205 - 210°C (colored in brown)

UV absorption spectrum $\lambda_{max}^{H_2O}$ 268 nm

$\lambda_{max}^{H_3O^+}$ 263 nm

Elemental analysis for $C_{14}H_{23}N_6O_7P$

Calculated : C, 40.20; H, 5.54; N, 20.09%

Measured : C, 39.87; H, 5.72; N, 19.78%

Preparation Example 9

Synthesis of $N^6$-(6-amino-n-hexyl)-3'-AMP

In 100 ml of water, 5.2 g of CRP-3',5'-P, 29 g of 1,6-diamino-n-hexane and 3 ml of triethylamine were dissolved, and the reaction was carried out at 80 to 90°C for 3 hours under stirring. The reaction mixture was treated similarly as in Preparation Example 8 and crystallized from water-ethanol to obtain 1.3 g of $N^6$-(6-amino-n-hexyl)-3'-AMP (Yield: 21%).

m.p. 240 - 245°C (decomposed)

UV absorption spectrum $\lambda_{max}^{H_2O}$ 268 nm

$\lambda_{max}^{H_3O^+}$ 263 nm

Elemental analysis for $C_{16}H_{27}N_6O_7P$

Calculated : C, 43.06; H, 6.10; N, 18.83%

Measured : C, 42.80; H, 6.33; N, 18.56%

Preparation Example 10

Synthesis of $N^6$-(8-amino-n-octyl)-3'-AMP

In 70 ml of water, 5.2 g of CRP-3',5'-P, 25 g of 1,8-diamino-n-octane and 3 ml of triethylamine were dissolved, and the reaction was carried out at 80 to 90° C for 3 hours. The reaction mixture was treated similarly as in Preparation Example 8 and crystallized from water-ethanol, whereupon 1.3 g of $N^6$-(8-amino-n-octyl)-3'-AMP (Yield: 19%) was obtained.

m.p. 210 - 213°C (decomposed)

UV absorption spectrum $\lambda_{max}^{H_2O}$ 268 nm

$\lambda_{max}^{H_3O^+}$ 263 nm

Elemental analysis for $C_{18}H_{31}N_6O_7P.H_2O$

Calculated : C, 43.92; H, 6.76; N, 17.07%

Measured : C, 44.28; H, 6.86; N, 16.86%

Preparation Example 11

Synthesis of $N^6$-(2-aminoethyl)-cAMP

In 50 ml of water, 3.1 g of CRP-3',5'-P and 10 ml of ethylenediamine were dissolved, and then 2 ml of triethylamine was added thereto. The reaction was carried out at 70 to 80°C for 3 hours. The reaction mixture was poured into one liter of water, then made acidic with hydrochloric acid, and adsorbed on a column of activated charcoal. Then, after washing with water, the column was eluted with a mixture of ethanol : conc.

aqueous ammonia: water (50 : 48 : 2). The eluted fractions containing the desired product were collected and stirred with addition of anion exchange resins, Dowex 1 x 2 (formic acid form). After the resins were separated by filtration, the filtrate was concentrated to dryness and crystallized from water-ethanol to obtain 2.2 g of $N^6$-(2-aminoethyl)-cAMP (Yield: 67%).

m.p. 275 - 280°C (decomposed)

UV absorption spectrum $\lambda_{max}^{H_2O}$ 267 nm

$\lambda_{max}^{H_3O^+}$ 263 nm

Elemental analysis for $C_{12}H_{17}N_6O_6P$

Calculated : C, 38.71; H, 4.60; N, 22.57%

Measured : C, 39.11; H, 4.83; N, 22.06%

<u>Preparation Example 12</u>

<u>Synthesis of $N^6$-(3-amino-n-propyl)-cAMP</u>

In 30 ml of water were dissolved 2.1 g of CRP-3',5'-P, 13 ml of 1,3-diamino-n-propane and 1.2 ml of triethylamine, and the reaction was carried out at 80°C for 25 hours. The reaction mixture was treated similarly as in Preparation Example 11 and crystallized from water-ethanol to obtain 1.6 g of $N^6$-(3-amino-n-propyl)-cAMP (Yield 71%).

m.p. 280 - 285°C (decomposed)

UV absorption spectrum $\lambda_{max}^{H_2O}$ 267 nm

$\lambda_{max}^{H_3O^+}$ 263 nm

Elemental analysis for $C_{13}H_{19}N_6O_6P$

Calculated : C, 40.42; H, 4.96; N, 21.76%

Measured : C, 40.03; H, 5.12; N, 22.09%

Preparation Example 13

Synthesis of $N^6$-(4-amino-n-butyl)-cAMP

In 100 ml of water, 5.2 g of CRP-3',5'-P, 24g of 1,4-diamino-n-butane and 3 ml of triethylamine were dissolved, and the reaction was carried out similarly as in Preparation Example 11. The reaction mixture was diluted with water, then made acidic with hydrochloric acid and adsorbed on a column of activated charcoal. Then, after washing with water, the column was subjected to elution with ethanol: aqueous ammonia: water (50 : 48 : 2). The eluate was applied to a column of Dowex 1 x 2 (formic acid form), and the fractions eluted with water were collected. This step was followed by lyophilization, whereupon 3.3 g of $N^6$-(4-amino-n-butyl)-cAMP (Yield 53%) was obtained.

m.p. 245 - 250°C (decomposed)

UV absorption spectrum $\lambda_{max}^{H_2O}$ 268 nm

$\lambda_{max}^{H_3O^+}$ 263 nm

Elemental analysis for $C_{14}H_{21}N_6O_6P.H_2O$

Calculated : C, 40.20; H, 5.54; N, 20.99%

Measured : C, 40.69; H, 5.42; N, 21.23%

Preparation Example 14

Synthesis of $N^6$-(6-amino-n-hexyl)-cAMP

In 100 ml of water, 5.2 g of CRP-3',5'-P, 29 g of 1,6-diamino-n-hexane and 3 ml of triethylamine were dissolved, and the reaction was carried out at 80 to 90°C for 3 hours under stirring. The reaction mixture was treated similarly as in Preparation Example 13 and crystallized from water-ethanol to obtain 3.2 g of $N^6$-(6-amino-n-hexyl)-cAMP (Yield: 51%).

m.p. 240 - 243°C (decomposed)

UV absorption spectrum $\lambda_{max}^{H_2O}$ 268 nm

$\lambda_{max}^{H_3O^+}$ 263.5 nm

Elemental analysis for $C_{16}H_{25}N_6O_6P$

Calculated : C, 43.06; H, 6.10; N, 18.83%

Measured : C, 42.80; H, 6.33; N, 18.56%

Preparation Example 15

Synthesis of $N^6$-(8-amino-n-octyl)-cAMP

In 70 ml of water, 5.2 g of CRP-3',5'-P, 25 g of 1,8-diamino-n-octane and 3 ml of triethylamine were dissolved, and the reaction was carried out at 80 to 90°C for 3 hours. The reaction mixture was treated similarly as in Preparation Example 13 and crystallized from ethanol, whereupon 4.1 g of $N^6$-(8-amino-n-octyl)-cAMP (Yield: 60%) was obtained.

m.p. 210 - 213°C (decomposed)

UV absorption spectrum $\lambda_{max}^{H_2O}$ 268 nm

$\lambda_{max}^{H_3O^+}$ 263 nm

Elemental analysis for $C_{18}H_{29}N_6O_6P$

Calculated : C, 47.38; H, 6.40; N, 18.42%

Measured : C, 47.06; H, 6.63; N, 18.04%

Preparation Example 16

Synthesis of $N^6$-(9-amino-n-nonyl)-cAMP

In 40 ml of water, 2.1 g of CRP-3',5'-P, 5.0g of 1,9-diamino-n-nonane and 2 ml of triethylamine were dissolved,and the reaction was carried out at 70 to 80°C for 6 hours. The reaction mixture was treated with a column of activated charcoal similarly as in Preparation Example 11 and the eluate was applied to a column of Dowex 1 x 2 (formic acid form). Elution was then carried out using a linear gradient of formic acid (0 → 0.04 M), and the eluted fractions containing the desired product were collected, concentrated, and crystallized from methanol-acetone to obtain 1.8 g of $N^6$-(9-amino-n-nonyl)-cAMP (Yield : 65%).

m.p. 206 - 208°C (decomposed)

UV absorption spectrum $\lambda_{max}^{H_2O}$ 268 nm

Elemental analysis for $C_{19}H_{31}N_6O_6P$

Calculated : C, 48.52; H, 6.64; N, 17.87%

Measured : C, 48.09; H, 6.78; N, 17.48%

Preparation Example 17

Synthesis of $N^6$-(10-amino-n-decanyl)-cAMP

In a mixture of 60 ml of water and 100 ml of dimethylformamide, 5.2 g of CRP-3',5'-P, 25 g of 1,10-diamino-n-decane and 3 ml of triethylamine were dissolved, and the reaction was carried out at 80 to 90°C for 5 hours. The reaction mixture was treated with a column of activated charcoal similarly as in Preparation Example 11, and the eluate was applied to a column of Dowex 1 x 2 (formic acid form). Elution was then carried out using a linear gradient of formic acid (0 → 0.15 M), and the eluted fractions containing the desired product were collected, concentrated and crystallized from methanol-acetone to obtain 4.2 g of $N^6$-(10-amino-n-decanyl)-cAMP (Yield: 59%).

m.p. 207 - 210°C (decomposed)

UV absorption spectrum $\lambda_{max}^{H_2O}$ 268 nm

Elemental analysis for $C_{20}H_{33}N_6O_6P$

Calculated: C, 49.60; H, 6.87; N, 17.35%

Measured : C, 49.93; H, 7.09; N, 17.00%

WHAT WE CLAIM IS :

1. An antiallergic, comprising an $N^6$-ω-aminoalkyl adenosine compound of the formula (I) or a pharmaceutically acceptable salt thereof as active ingredient:

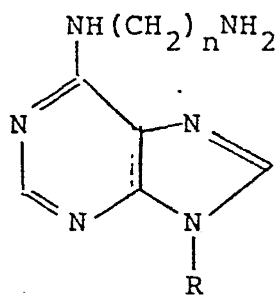

(I),

wherein n is an integer of 1 to 20, and R is a member selected from the group consisting of:

a β-D-ribofuranosyl group represented by the formula

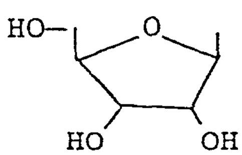

;

a 5-phosphoryl-β-D-ribofuranosyl group of the formula

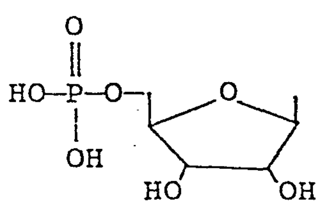

;

a 3-phosphoryl-β-D-ribofuranosyl group of the formula

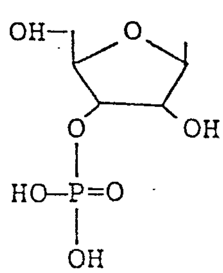

; and

a 3,5-cyclicphosphoryl-β-D-ribofuranosyl group of the formula

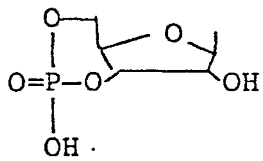

2. An antiallergic as set forth in claim 1 comprising an $N^6$-ω-aminoalkyl adenosine compound of the formula (I) wherein n is an integer of 6 to 20, and R is the β-D-ribofuranosyl group, or a pharmaceutically acceptable salt thereof.

3. An antiallergic as set forth in claim 1 comprising an $N^6$-ω-aminoalkyl adenosine compound of the formula (I) wherein n is an integer of 13 to 20 and R is the 5-phosphoryl-β-D-ribofuranosyl group, or a pharmaceutically acceptable salt thereof.

4. An antiallergic as set forth in claim 1 comprising an $N^6$-ω-aminoalkyl adenosine compound of the formula (I) wherein n is an integer of 1 to 20 and R is the 3-phosphoryl-β-D-ribofuranosyl group, or a pharmaceutically acceptable salt thereof.

5. An antiallergic as set forth in claim 1 comprising an $N^6$-ω-aminoalkyl adenosine compound of the formula (I) wherein n is an integer of 1 to 20 and R is the 3,5-cyclicphosphoryl-β-D-ribofuranosyl group, or a pharmaceutically acceptable salt thereof.

6. An antiallergic as set forth in any of claims 1 to 5 wherein the pharmaceutically acceptable salt is a salt of the $N^6$-ω-aminoalkyl adenosine compound with a member selected from the group consisting of alkali metals, alkaline earth metals, ammonia, mineral acids, organic acids and amino acids.

7. A pharmaceutical composition useful in the treatment of allergic disorder comprising a safe and effective amount of an antiallergic as set forth in any of claims 1 to 6, and a pharmaceutically acceptable carrier.

8. A method for treating allergic disorder comprising administering to a human or animal in need of such treatment a safe and effective amount of the antiallergic as claimed in any of claims 1 to 6.

9. A method as set forth in claim 8 wherein the antiallergic is administered at a dosage level of 0.01 to 10 mg/kg-body weight of the human or animal.

European Patent Office

# EUROPEAN SEARCH REPORT

0061001

Application number

EP 82 10 1303

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 82, no. 1, 6th January 1975, page 326, no. 325c, Columbus Ohio (USA); SHIKITA, MIKIO et al.: "Hypotensive and radioprotective properties of N6-substituted adenosine derivatives" & CHEM. PHARM. BULL. 1974, 22(6),1410-13 *Abstract and chemical abstracts ninth collective index 1972-1976, chemical substances A-ames p849CS: adenosine, -,N-(3-aminopropyl)* --- | 1,4-9 | A 61 K 31/70 // C 07 H 19/16 C 07 H 19/20 |
| X | CHEMICAL ABSTRACTS, vol. 88, no. 23, 5th June 1978, page 238, no. 165931j, Columbus Ohio (USA); E.RIEKE et al.: "Mechanism of activation of protein kinase I from rabbit skeletal muscle. 3. Investigation with agarose-immobilized cAMP derivatives" & EUR. J. BIOCHEM. 1978, 83(2),pages 419-426. "Abstract and chemical abstracts, chemical substance index, A-B, vol. 88, January-June 1978, page 106CS, abstract and adenosine, -,N-(2-Aminoethyl) -cyclic 3',5'-(hydrogen phosphate) and adenosine, -,N-(2-aminohexyl)-cyclic 3',5'-(hydrogen phosphate)* --- | 1-9 | TECHNICAL FIELDS SEARCHED (Int. Cl. 3) A 61 K 31/00 |

The present search report has been drawn up for all claims

| Place of search | Date of completion of the search | Examiner |
|---|---|---|
| THE HAGUE | 26-04-1982 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0061001

Application number

EP 82 10 1303

Page 2

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| P,X | BIOCHEMISTRY, vol. 20, no. 8, 14th April 1981, pages 2245-2251, American Chemical Society, USA; R.G.KEMP: "Interaction of dinucleotides with muscle phosphofructokinase". *Page 2249, last all* ----- | 1-9 | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 26-04-1982 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82